# EUROPEAN PATENT SPECIFICATION

(11) **EP 3 463 105 B1**
(45) Date of publication and mention of the grant of the patent: **06.11.2024**
(21) Application number: 17807579.2
(22) Date of filing: 02.06.2017
(51) Int. Cl.: A61B 17/00, A61B 17/16, A61B 17/56, A61B 17/68, A61B 17/70, A61B 17/88, A61B 17/72, A61B 17/86

(54) **DIFFERENTIAL COMPRESSION BONE SCREW**
KNOCHENSCHRAUBE MIT DIFFERENTIELLER KOMPRESSION
VIS OSSEUSE À COMPRESSION DIFFÉRENTIELLE

(30) Priority: 02.06.2016 US 201662344823 P
(43) Date of publication of application: 10.04.2019
(73) Proprietor: In2Bones USA, LLC, Memphis, TN 38119 (US); Varner, Kevin E., Memphis, TN 38119 (US); Heier, Keith A., Memphis, TN 38119 (US); Hanson, Travis W., Memphis, TN 38119 (US); Chambers, Casey M., Memphis, TN 38119 (US); Wahl, Rebecca, Hawkins, Escondido, CA 92025 (US)
(72) Inventor: VARNER, Kevin, E., Memphis, TN 38119 (US); HEIER, Keith, A., Memphis, TN 38119 (US); HANSON, Travis, W., Memphis, TN 38119 (US); CHAMBERS, Casey, M., Memphis, TN 38119 (US); WAHL, Rebecca, Hawkins, Escondido, CA 92025 (US)
(74) Representative: Dehns
(86) International application number: PCT/US2017/035711
(87) International publication number: WO 2017/210574

(56) References cited:
- WO-A1-2007/107995
- WO-A1-2015/100149
- WO-A2-2016/007415
- US-A- 6 030 162
- US-A- 6 123 711
- US-A1- 2006 025 772
- US-A1- 2012 022 603
- US-B1- 6 306 140
- US-B2- 6 969 390
- US-B2- 8 414 628
- US-B2- 9 161 793

## Description

### FIELD

The field of the present disclosure generally relates to securing bones together. More particularly, the field of the present disclosure relates to bone screws according to the appended claims.

### BACKGROUND

A fusion bone plate implant may be utilized in conjunction with one or more fasteners so as to generate compression and stability at a bone interface. An implant coupled with fasteners generally serves to stabilize bones, or bone parts, relative to one another so as to promote bone fusion. In many applications, bone plates and fasteners are used to fuse bones, or bone parts, of the human body, such as bones in the foot, the ankle, the hand, the wrist, as well as various other portions of the body. Furthermore, during the course of certain medical procedures, a surgeon may immobilize one or more bones or the bone fragments by stabilizing the bones together in a configuration which approximates the natural anatomy. To this end, the surgeon may use fasteners to attach the bones to a bone plate implant so as to hold the bones in alignment with one another while they fuse together.

In some instances, however, a bone plate may be impractical for implantation in a portion of the body that requires treatment. What is needed, therefore, is a compression bone screw that is configured to fuse bones in absence of a fusion bone plate.

A bone screw is disclosed in US6030162A. Document US6306140B1 discloses a bone screw according to the preamble of claim 1.

### SUMMARY

A compression bone screw is provided as defined by claim 1.

### BRIEF DESCRIPTION OF THE DRAWINGS

The drawings refer to embodiments of the present disclosure in which:
Figure 1 illustrates an isometric view of a distal portion of an exemplary embodiment of a compression bone screw that may be used for repairing bones of a patient;
Figure 2 illustrates an isometric view of a proximal portion of an exemplary embodiment of a compression bone screw that may be used for repairing bones of a patient;
Figure 3 illustrates a side view of an exemplary embodiment of a compression bone screw that may be used for repairing bones of a patient;
Figure 4 illustrates a side view of the exemplary embodiment of the compression bone screw of Fig. 3, showing exterior diameters along a shank of the compression bone screw;
Figure 5 illustrates an exemplary use environment wherein an exemplary embodiment of a differential compression bone screw is longitudinally disposed within substantially the center of a repaired bone;
Figure 6 illustrates an isometric view of a proximal portion of an exemplary embodiment of a compression bone screw that may be used for repairing bones of a patient; and
Figure 7 illustrates a side view of the exemplary embodiment of the compression bone screw of Fig. 6, showing a differential thread pitch disposed along a shank of the compression bone screw.

While the present disclosure is subject to various modifications and alternative forms, specific embodiments thereof have been shown by way of example in the drawings and will herein be described in detail. The invention should be understood to not be limited to the particular forms disclosed, but on the contrary, the intention is to cover all modifications, and alternatives falling within the scope of the invention as defined by the claims.

### DETAILED DESCRIPTION

In the following description, numerous specific details are set forth in order to provide a thorough understanding of the present disclosure. It will be apparent, however, to one of ordinary skill in the art that the invention disclosed herein may be practiced without these specific details. In other instances, specific numeric references such as "first screw," may be made. However, the specific numeric reference should not be interpreted as a literal sequential order but rather interpreted that the "first screw" is different than a "second screw." Thus, the specific details set forth are merely exemplary. The specific details may be varied from and still be contemplated to be within the scope of the invention as claimed. The term "coupled" is defined as meaning connected either directly to the component or indirectly to the component through another component. Further, as used herein, the terms "about," "approximately," or "substantially" for any numerical values or ranges indicate a suitable dimensional tolerance that allows the part or collection of components to function for its intended purpose as described herein.

In general, the present disclosure describes an apparatus and a method for a differential compression bone screw for compressing two adjacent bone portions together, including compressing bone fractures, fixating osteotomies, and joining fusions. The compression bone screw is comprised of a head portion and a distally extending shank. A center hole extends from the head portion to a distal end of the shank. The head portion is comprised of a superior end and an inferior end. The superior end includes a shaped opening that is substantially concentric with the center hole and configured to engagedly receive a tool for driving the compression bone screw into a hole drilled in a patient's bone. The inferior end includes a plurality of barbs that are disposed around the circumference of the inferior end and are configured to fixedly engage with surrounding bone tissue. Proximal threads and distal threads are disposed on the shank and configured to rotatably engage within the hole in the patient's bone, such that the compression bone screw advances into the hole upon being turned by way of the tool. A thread pitch of the distal threads preferably is greater than a thread pitch of the proximal threads, such that the compression bone screw comprises a differential pitch configured to compress the two adjacent bone portions, thereby closing a fracture there between.

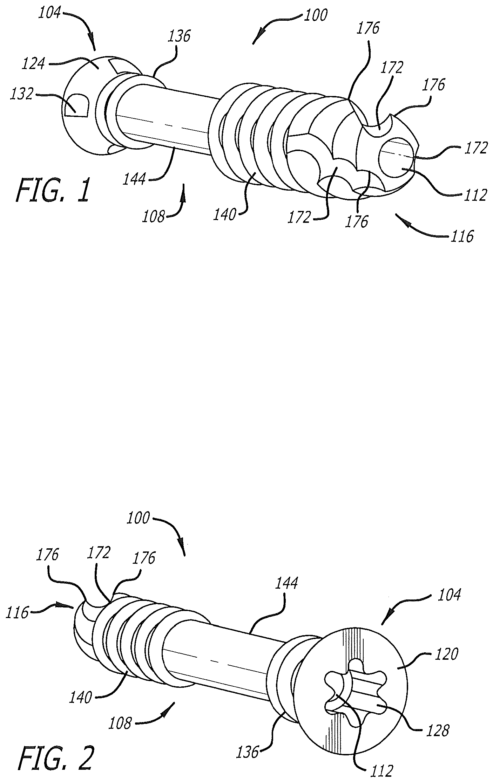

Figures 1-4 illustrate an exemplary embodiment of a compression bone screw 100 that may be used for repairing bone fractures, fixating osteotomies, joining fusions of the skeletal system, and the like. It should be understood that the terms "bone screw," "fastener," "fixator," "elongate member," and "screw" may be used interchangeably herein as they essentially describe the same type of device. The compression bone screw 100 generally is an elongate member comprised of a head portion 104 and a shank 108. As best shown in Figs. 1-2, a cannulation or center hole 112 extends longitudinally from the head portion 104 to a distal end 116 of the shank 108. The center hole 112 is configured to receive any of various guidewires, trocars, and other similar instruments for directing the bone screw to a hole drilled in the patient's bone,

The head portion 104 is comprised of a superior end 120 and an inferior end 124. As best illustrated in Fig. 2, the superior end 120 may include a shaped opening 128 that is substantially concentric with the center hole 112. The shaped opening 128 generally is configured to engagedly receive a tool suitable for driving the bone screw 100 into the hole drilled in the patient's bone. Although in the illustrated embodiment, the shaped opening 128 is comprised of a hexalobe shape, any of various multi-lobe shapes, as well as other polygonal shapes, are also contemplated.

The inferior end 124 preferably is configured to countersink within the hole in the bone. Thus, the superior end 120 is not left protruding above the exterior surface of the bone once the compression bone screw 100 is fully engaged with the patient's bone. Further, a plurality of barbs 132 disposed around the circumference of the inferior end 124 are configured to engage with the surrounding bone tissue so as to prevent the bone screw from backing out of the hole in the patient's bone. In some embodiments, however, the inferior end 124 may be configured to be received within an opening of a bone fusion plate, such that the superior end 120 countersinks within the opening of the bone fusion plate and presses the plate against the surface of the patient's bone.

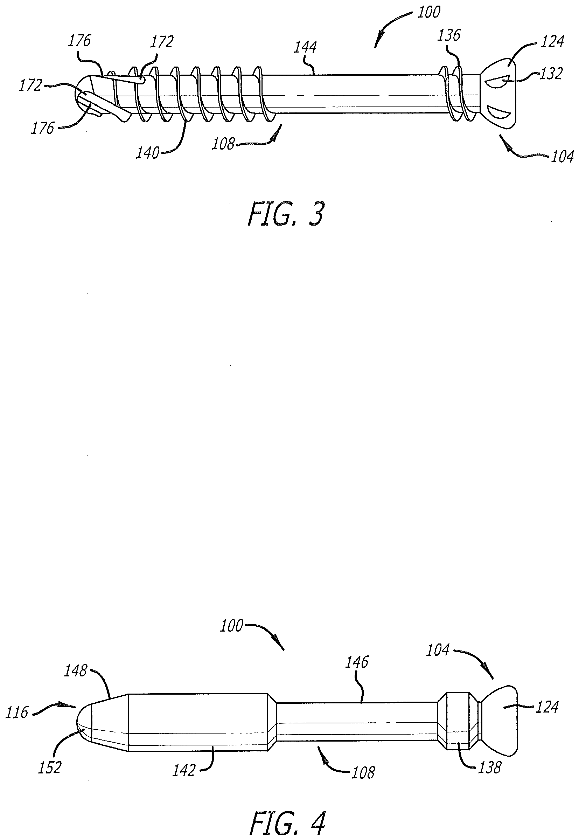

As best shown in Figs. 3-4, the shank 108 is comprised of proximal threads 136 and distal threads 140 that share an intervening smooth portion 144. The proximal threads 136 have a diameter 138, and the distal threads 140 have a diameter 142. A tapered diameter 148 extends from the distal threads 140 to a rounded portion 152 that comprises the distal end 116. The smooth portion 144 is comprised of a diameter 146 that is less than the diameters 138 and 142 so as to facilitate passing the smooth portion 144 through the bone with relatively little resistance. Further, in the illustrated embodiment, the threads 136 and 140 share substantially similar exterior diameters, 138 and 142, respectively, as shown in Fig. 4. In some embodiments, however, the diameter 138 of the proximal threads 136 may be greater than the diameter 142 of the distal threads 140. Various diameters of the proximal threads 136 and the distal threads 140, as well as the diameter 146, are contemplated, without limitation.

The threads 136 and 140 are configured to rotatably engage within a suitably sized hole drilled in the patient's bone. Thus, turning the bone screw 100 in an appropriate direction by way of a tool coupled with the shaped opening 128, drives the distal threads 140 to engage with bone tissue surrounding the hole, and thus advancing the bone screw 100 deeper into the hole in the bone. The proximal threads 136 engage the bone once a majority of the bone screw 100 is already disposed within the hole in the bone. Continued turning of the bone screw 100 then countersinks the inferior end 124 into an upper-most portion of the hole in the bone, and draws the superior end 120 beneath the exterior surface of the patient's bone.

Moreover, the illustrated embodiment of the compression bone screw 100 comprises a differential pitch wherein the distal threads 140 have a thread pitch that is greater than the thread pitch of the proximal threads 136. In operation, the greater thread pitch of the distal threads 140 pushes the bone portion near the distal threads toward the bone portion near the proximal threads 136. The diameter 146 of the smooth portion 144 allows the fracture to close as the bone portions are compressed together. In some embodiments, the thread pitch of the distal threads 140 may range between substantially 1-3 times greater than the thread pitch of the proximal threads 136. Preferably, however, the thread pitch of the distal threads 140 is substantially 2-times greater than the thread pitch of the proximal threads 136. A wide variety of differential pitch configurations are contemplated within the scope of the present disclosure.

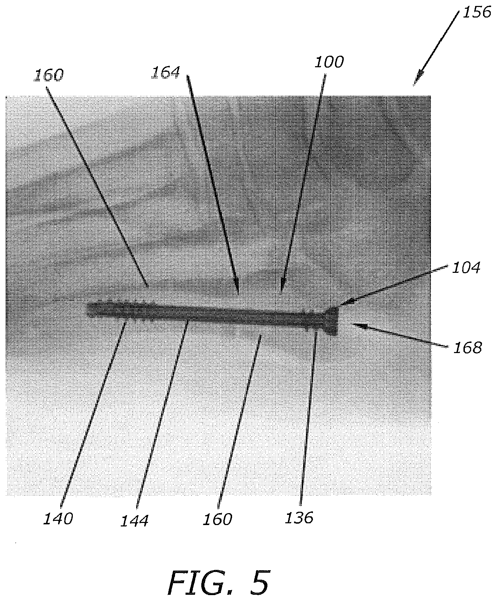

It is contemplated that the differential pitch of the compression bone screw 100 is particularly well suited for compressing bone fractures, fixating osteotomies, joining fusions, as well as any other surgical procedure wherein compressing two adjacent bone portions is desired, without limitation. It is further contemplated that the compression bone screw 100 may be advantageously oriented longitudinally with respect to a patient's bone. Figure 5 illustrates an exemplary use environment 156 wherein the compression bone screw 100 is longitudinally disposed within substantially the center of a repaired bone 160. The proximal threads 136 and the distal threads 140 are engaged with healthy bone tissue, while a repaired fracture 164 is disposed along the smooth portion 144. Further, the head portion 104 is countersunk within an entry hole 168 that was drilled into the repaired bone 160 by a surgeon. As will be appreciated, the compression bone screw 100 may be implemented in any of various lengths and diameters so as to advantageously repair a wide variety of differently sized and shaped bones within the human body. Furthermore, it is envisioned that the compression bone screw 100 may be configured for use in a veterinary capacity, and thus the bone screw 100 may be implemented with various shapes and sizes that are suitable for use in different types of animals.

As will be appreciated, the rounded portion 152 and the tapered diameter 148 are configured to minimize resistance to forward movement of the compression bone screw 100 advancing within the interior of a bone hole. As best shown in Fig. 1, the distal end 116 and the tapered diameter 148 of the bone screw 100 are further comprised of one or more flutes 172 that extend from adjacent of the center hole 112 and spiral along the tapered diameter 148. A pair of cutting edges 176 borders each of the flutes 172. Although the illustrated embodiment of the bone screw 100 comprises three flutes 172, and thus six cutting edges 176, more than or less than three flutes 172 and six cutting edges 176 may be incorporated into different implementations of the bone screw 100 without limitation. As will be appreciated, the cutting edges 176 advantageously clean the interior of the bone hole and increase the diameter of the hole to accept the distal threads 140 of the advancing bone screw 100. As will be appreciated, the spiral, or a rate of twist, of the flutes 172 generally controls the rate of bone debris removal from the interior of the bone hole during rotation of the bone screw 100. It is contemplated that the flutes 172 may be implemented with any of various spirals without deviating beyond the scope of the claims.

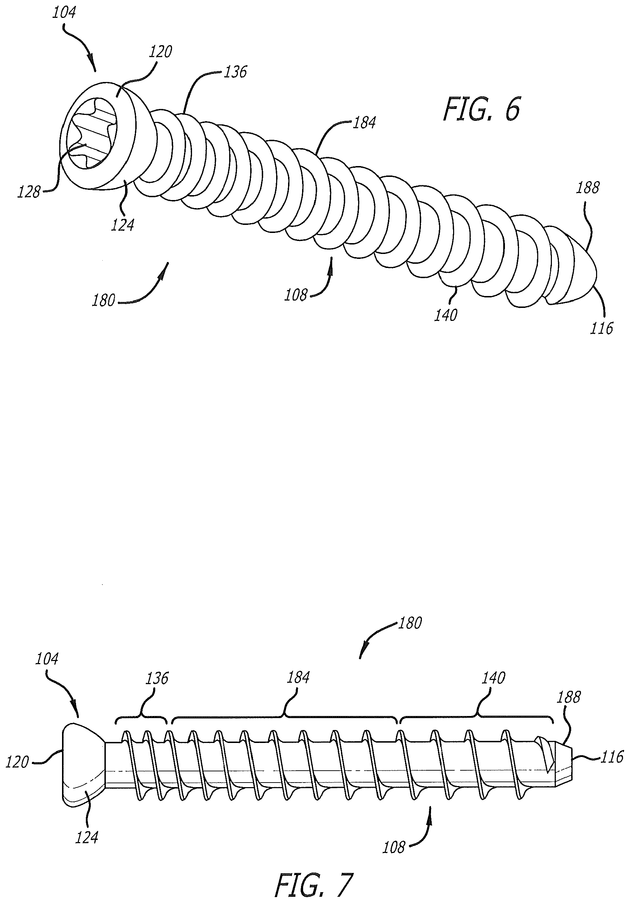

Figures 6-7 illustrate an exemplary embodiment of a compression bone screw 180 that may be used for repairing bones of a patient. The compression bone screw 180 is substantially similar to the compression bone screw 100, illustrated in Figs. 1-3, with the exception that the compression bone screw 180 is comprised of intermediate threads 184 in lieu of the smooth portion 144. As best shown in Fig. 7, the intermediate threads 184 extend from the distal threads 140 to the proximal threads 136, such that a continuous series of threads are disposed along substantially an entirety of the shank 108. Further, a tapered diameter 188 extends from the distal threads 140 to the distal end 116. The tapered diameter 188 and the distal end 116 are configured to minimize resistance to forward movement of the compression bone screw 180 advancing within the interior of a bone hole. It is contemplated that, in some embodiments, the tapered diameter 188 may be further comprised of one or more flutes 172 and cutting edges 176 that extend from the distal end 116 to the distal threads 140, as described herein.

The intermediate threads 184 may have a thread pitch that generally changes along the length of the shank 108. In the illustrated embodiment of Figs. 6-7, the intermediate threads 184 have a thread pitch that continuously decreases from the thread pitch of the distal threads 140 to the relatively smaller thread pitch of the proximal threads 136. In some embodiments, the intermediate threads 184 may be comprised of a thread pitch near the distal threads 140 that ranges between substantially 1-3 times greater than the thread pitch of the intermediate threads that are near the proximal threads 136. In some embodiments, the intermediate threads 184 may be comprised of a thread pitch near the distal threads 140 that decreases from substantially 2-times greater than the thread pitch near the proximal threads 136. During operation of the compression bone screw 180, the greater thread pitch of the distal threads 140 and nearby intermediate threads 184 pushes the bone portion near the distal threads toward the bone portion near the proximal threads 136. As will be appreciated, during operation of the compression bone screw 180, the decreasing thread pitch of the intermediate threads 184 contributes to compressing the bone portion near the distal threads 140 toward the bone portion near the proximal threads 136. A wide variety of differential pitch configurations are contemplated within the scope of the present disclosure.

Moreover, in some embodiments, wherein the proximal threads 136 have a larger diameter than the distal threads 140, the intermediate threads 184 may be comprised of a diameter that continuously increases from the diameter of the distal threads 140 to the diameter of the proximal threads 136. In some embodiments, the intermediate threads 184 may have a diameter that is larger than the diameter of the distal threads 140 and is smaller than the diameter of the proximal threads 136. In still some embodiments, the diameter of the intermediate threads 184 may be substantially the same as the diameter of the distal threads 140 along a majority of the intermediate threads and then abruptly increase to match the diameter of the proximal threads 136. It should be understood, therefore, that a wide variety of relationships between the shapes and sizes of the distal threads 140, the intermediate threads 184, and the proximal threads 136 are contemplate and may be implemented within the scope of the claims.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. The scope of the invention is to be understood as not limited by the specific embodiments described herein, but only by scope of the appended claims.

## Claims

1. A compression bone screw for compressing adjacent bone portions together, comprising:
a head portion (104) comprised of a superior end (120) and an inferior end (124);
a shank (108) extending distally from the inferior end to a distal end (116); and
proximal threads (136) and distal threads (140) disposed along the shank, the proximal threads comprised of a first thread pitch and the distal threads comprised of a second thread pitch, such that the shank comprises a differential pitch; wherein
the superior end includes a shaped opening (128) that is substantially concentric with the head portion and configured to engagedly receive a tool for driving the compression bone screw into the hole drilled in the bone, and wherein the inferior end comprises a plurality of barbs disposed around the circumference of the inferior end and configured to fixedly engage with surrounding bone tissue; and wherein the shaped opening (128) comprises a multi-lobe shape;
wherein a center hole (112) extends from the head portion to the distal end and is configured to receive any of various guidewires, trocars, and other similar instruments configured to direct the compression bone screw to a target opening of a hole drilled in a bone;
**characterised in that**:
a tapered diameter (148) extends from the distal threads (140) to a rounded portion (152) that comprises the distal end; and **in that** the distal end (116) and the tapered diameter (148) of the bone screw (100) are further comprised of one or more flutes (172) that extend from adjacent of the center hole (112) and spiral along the tapered diameter (148); wherein a pair of cutting edges (176) borders each of the flutes (172).

2. The bone screw of claim 1, wherein intermediate threads (184) are disposed between the distal threads and the proximal threads and comprise a thread pitch that decreases from the second thread pitch near the distal threads to the first thread pitch near the proximal threads.

3. The bone screw of any preceding claim, wherein the inferior end is configured to engage within an opening of the hole drilled in the bone, such that the superior end countersinks below the exterior surface of the bone, or wherein the inferior end is configured to engage within an opening of a bone fusion plate, such that the superior end countersinks within the opening and the inferior end presses the bone fusion plate against the surface of the bone.

4. The bone screw of any preceding claim, wherein the distal threads and the proximal threads are configured to rotatably engage within a hole drilled in a bone, such that the compression bone screw advances into the hole upon being turned by way of a suitable tool, and preferably wherein the proximal threads comprise a first diameter, the distal threads comprise a second diameter, and a smooth portion (144) disposed between the proximal threads and the distal threads is comprised of a diameter that is less than the first diameter and the second diameter, and wherein the first diameter is greater than the second diameter, and further preferably wherein the rounded portion and the tapered diameter are configured to minimize resistance to forward movement of the compression bone screw advancing within the interior of a hole drilled in a bone, and/or further preferably wherein each pair cutting edges is configured to clean the interior of a hole drilled in a bone during advancing of the compression bone screw within the hole.

5. The bone screw of any preceding claim, wherein the second thread pitch is greater than the first thread pitch, such that the distal threads push a first bone portion toward a second bone portion near the proximal threads, thereby closing a fracture between the first bone portion and the second bone portion.

6. The bone screw of claim 5, wherein a smooth portion disposed between the proximal threads and distal threads is configured to allow the fracture to close as the first bone portion and the second bone portion are compressed together.

7. The bone screw of claim 5 or 6, wherein the second thread pitch ranges between substantially 1 to 3 times greater than the first thread pitch, and preferably wherein the second thread pitch is substantially 2-times greater than the first thread pitch.

8. The bone screw of claim 5, 6 or 7, wherein intermediate threads are disposed between the distal threads and the proximal threads, the intermediate threads having a thread pitch that decreases from the second thread pitch near the distal threads to the first thread pitch near the proximal threads.

## Patentansprüche

1. Kompressionsknochenschraube zum Zusammendrücken benachbarter Knochenabschnitte, umfassend:
einen Kopfabschnitt (104), der ein oberes Ende (120) und ein unteres Ende (124) umfasst;
einen Schaft (108), der sich distal vom unteren Ende zu einem distalen Ende (116) erstreckt; und
proximale Gewindegänge (136) und distale Gewindegänge (140), die entlang des Schafts angeordnet sind, wobei die proximalen Gewindegänge eine erste Gewindesteigung umfassen und die distalen Gewindegänge eine zweite Gewindesteigung umfassen, so dass der Schaft eine unterschiedliche Steigung aufweist; wobei
das obere Ende eine geformte Öffnung (128) einschließt, die im Wesentlichen konzentrisch zum Kopfabschnitt ist und so konfiguriert ist, dass sie ein Werkzeug zum Eintreiben der Kompressionsknochenschraube in das im Knochen gebohrte Loch im Eingriff aufnimmt, und wobei das untere Ende eine Vielzahl von um den Umfang des unteren Endes herum angeordnete Widerhaken umfasst und so konfiguriert, dass es fest mit dem umgebenden Knochengewebe in Eingriff kommt; und wobei die geformte Öffnung (128) eine mehrlappige Form umfasst;
wobei sich ein Mittelloch (112) vom Kopfabschnitt bis zum distalen Ende erstreckt und so konfiguriert ist, dass es beliebige von verschiedenen Führungsdrähten, Trokaren und anderen ähnlichen Instrumenten aufnimmt, die so konfiguriert sind, dass sie die Kompressionsknochenschraube zu einer Zielöffnung eines in einen Knochen gebohrten Lochs führen;
**dadurch gekennzeichnet, dass**:
ein verjüngter Durchmesser (148) sich von den distalen Gewindegängen (140) zu einem abgerundeten Abschnitt (152) erstreckt, der das distale Ende umfasst; und dass das distale Ende (116) und der verjüngte Durchmesser (148) der Knochenschraube (100) weiter eine oder mehrere Rillen (172) umfassen, die sich angrenzend an das Mittelloch (112) und spiralförmig entlang des verjüngten Durchmessers (148) erstrecken; wobei ein Paar Schneidkanten (176) jede der Rillen (172) begrenzt.

2. Knochenschraube nach Anspruch 1, wobei Zwischengewinde (184) zwischen den distalen Gewindegängen und den proximalen Gewindegängen angeordnet sind und eine Gewindesteigung umfassen, die von der zweiten Gewindesteigung in der Nähe der distalen Gewindegänge zu der ersten Gewindesteigung in der Nähe der proximalen Gewindegänge abnimmt.

3. Knochenschraube nach einem vorstehenden Anspruch, wobei das untere Ende so konfiguriert ist, dass es in eine Öffnung des in den Knochen gebohrten Lochs eingreift, so dass das obere Ende unter die Außenfläche des Knochens versinkt, oder wobei das untere Ende so konfiguriert ist, dass es in einer Öffnung einer Knochenfusionsplatte in Eingriff kommt, so dass das obere Ende in der Öffnung versinkt und das untere Ende die Knochenfusionsplatte gegen die Oberfläche des Knochens drückt.

4. Knochenschraube nach einem vorstehenden Anspruch, wobei die distalen Gewindegänge und die proximalen Gewindegänge so konfiguriert sind, dass sie drehbar in ein in einen Knochen gebohrtes Loch eingreifen, so dass die Kompressionsknochenschraube beim Drehen mit einem geeigneten Werkzeug in das Loch vordringt, und wobei bevorzugt die proximalen Gewindegänge einen ersten Durchmesser umfassen, die distalen Gewindegänge einen zweiten Durchmesser umfassen, und ein glatter Abschnitt (144), der zwischen den proximalen Gewindegängen und den distalen Gewindegängen angeordnet ist, einen Durchmesser umfasst, der kleiner ist als der erste Durchmesser und der zweite Durchmesser, und wobei der erste Durchmesser größer ist als der zweite Durchmesser, und wobei der abgerundete Abschnitt und der verjüngte Durchmesser weiter bevorzugt so konfiguriert sind, dass sie den Widerstand gegen eine Vorwärtsbewegung der Kompressionsknochenschraube, die im Inneren eines in einen Knochen gebohrten Lochs vordringt, minimieren, und /oder wobei jedes Schneidkantenpaar weiter bevorzugt so konfiguriert ist, dass es das Innere eines in einen Knochen gebohrten Lochs reinigt, während die Kompressionsknochenschraube innerhalb des Lochs vorgeschoben wird.

5. Knochenschraube nach einem vorstehenden Anspruch, wobei die zweite Gewindesteigung größer ist als die erste Gewindesteigung, so dass die distalen Gewindegänge einen ersten Knochenabschnitt in Richtung eines zweiten Knochenabschnitts in der Nähe der proximalen Gewindegänge drücken und dadurch einen Bruch zwischen dem ersten Knochenabschnitt und dem zweiten Knochenabschnitt schließen.

6. Knochenschraube nach Anspruch 5, wobei ein glatter Abschnitt, der zwischen den proximalen Gewindegängen und den distalen Gewindegängen angeordnet ist, so konfiguriert ist, dass er ein Schließen der Fraktur ermöglicht, wenn der erste Knochenabschnitt und der zweite Knochenabschnitt zusammengedrückt werden.

7. Knochenschraube nach Anspruch 5 oder 6, wobei die zweite Gewindesteigung im Wesentlichen 1 bis 3 Mal so groß ist wie die erste Gewindesteigung und wobei die zweite Gewindesteigung bevorzugt im Wesentlichen 2 Mal so groß ist wie die erste Gewindesteigung.

8. Knochenschraube nach Anspruch 5, 6 oder 7, wobei Zwischengewindegänge zwischen den distalen Gewindegängen und den proximalen Gewindegängen angeordnet sind, wobei die Zwischengewindegänge eine Gewindesteigung aufweisen, die von der zweiten Gewindesteigung in der Nähe der distalen Gewindegänge zu der ersten Gewindesteigung in der Nähe der proximalen Gewindegänge abnimmt.

## Revendications

1. Vis osseuse à compression pour comprimer ensemble des parties osseuses adjacentes, comprenant :
une partie de tête (104) composée d'une extrémité supérieure (120) et d'une extrémité inférieure (124) ;
une tige (108) s'étendant distalement de l'extrémité inférieure à une extrémité distale (116) ; et
des filetages proximaux (136) et des filetages distaux (140) disposés le long de la tige, les filetages proximaux étant composés d'un premier pas de filetage et les filetages distaux étant composés d'un second pas de filetage, de sorte que la tige comprenne un pas différentiel ; dans laquelle
l'extrémité supérieure inclut une ouverture façonnée (128) qui est sensiblement concentrique avec la partie de tête et configurée pour recevoir en prise un outil pour enfoncer la vis osseuse à compression dans le trou percé dans l'os, et dans laquelle l'extrémité inférieure comprend une pluralité de barbes disposées sur la circonférence de l'extrémité inférieure et configurées pour venir en prise à demeure avec le tissu osseux environnant ; et dans laquelle l'ouverture façonnée (128) comprend une forme multilobée ;
dans laquelle un trou central (112) s'étend de la partie de tête à l'extrémité distale et est configuré pour recevoir l'un quelconque de divers fils-guides, trocarts et autres instruments similaires configurés pour diriger la vis osseuse à compression vers une ouverture cible d'un trou percé dans un os ;
**caractérisée en ce que** :
un diamètre effilé (148) s'étend des filetages distaux (140) à une partie arrondie (152) qui comprend l'extrémité distale ; et **en ce que** l'extrémité distale (116) et le diamètre effilé (148) de la vis osseuse (100) sont composés en outre d'une ou plusieurs cannelures (172) qui s'étendent depuis une position adjacente au trou central (112) et s'enroulent en spirale le long du diamètre effilé (148) ; dans laquelle une paire d'arêtes coupantes (176) borde chacune des cannelures (172).

2. Vis osseuse selon la revendication 1, dans laquelle des filetages intermédiaires (184) sont disposés entre les filetages distaux et les filetages proximaux et comprennent un pas de filetage qui diminue du second pas de filetage près des filetages distaux au premier pas de filetage près des filetages proximaux.

3. Vis osseuse selon une quelconque revendication précédente, dans laquelle l'extrémité inférieure est configurée pour venir en prise dans une ouverture du trou percé dans l'os, de sorte que l'extrémité supérieure fraise sous la surface extérieure de l'os, ou dans laquelle l'extrémité inférieure est configurée pour venir en prise à l'intérieur d'une ouverture d'une plaque de fusion osseuse, de sorte que l'extrémité supérieure fraise dans l'ouverture et que l'extrémité inférieure presse la plaque de fusion osseuse contre la surface de l'os.

4. Vis osseuse selon une quelconque revendication précédente, dans laquelle les filetages distaux et les filetages proximaux sont configurés pour venir en prise de manière rotative dans un trou percé dans un os, de sorte que la vis osseuse à compression avance dans le trou lorsqu'elle est tournée au moyen d'un outil approprié, et de préférence dans laquelle les filetages proximaux comprennent un premier diamètre, les filetages distaux comprennent un second diamètre, et une partie lisse (144) disposée entre les filetages proximaux et les filetages distaux est composée d'un diamètre qui est inférieur au premier diamètre et au second diamètre, et dans laquelle le premier diamètre est supérieur au second diamètre, et en outre de préférence dans laquelle la partie arrondie et le diamètre effilé sont configurés pour réduire au minimum la résistance au mouvement vers l'avant de la vis osseuse à compression avançant à l'intérieur d'un trou percé dans un os, et/ou en outre de préférence dans laquelle chaque paire d'arêtes coupantes est configurée pour nettoyer l'intérieur d'un trou percé dans un os pendant l'avancement de la vis osseuse à compression à l'intérieur du trou.

5. Vis osseuse selon une quelconque revendication précédente, dans laquelle le second pas de filetage est supérieur au premier pas de filetage, de sorte que les filetages distaux poussent une première partie osseuse vers une seconde partie osseuse à proximité des filetages proximaux, fermant ainsi une fracture entre la première partie osseuse et la seconde partie osseuse.

6. Vis osseuse selon la revendication 5, dans laquelle une partie lisse disposée entre les filetages proximaux et les filetages distaux est configurée pour permettre à la fracture de se fermer lorsque la première partie osseuse et la seconde partie osseuse sont comprimées ensemble.

7. Vis osseuse selon la revendication 5 ou 6, dans laquelle le second pas de filetage est sensiblement compris entre 1 à 3 fois plus grand que le premier pas de filetage, et de préférence dans laquelle le second pas de filetage est sensiblement 2 fois plus grand que le premier pas de filetage.

8. Vis osseuse selon la revendication 5, 6 ou 7, dans laquelle des filetages intermédiaires sont disposés entre les filetages distaux et les filetages proximaux, les filetages intermédiaires présentant un pas de filetage qui diminue du second pas de filetage près des filetages distaux au premier pas de filetage près des filetages proximaux.
